# EUROPÄISCHE PATENTSCHRIFT

(11) **EP 3 321 205 B1**
(45) Veröffentlichungstag und Bekanntmachung des Hinweises auf die Patenterteilung: **05.06.2019**
(21) Anmeldenummer: 17201357.5
(22) Anmeldetag: 13.11.2017
(51) Int. Cl.: B65D 21/02, B65D 21/06, B65D 6/08

(54) **STAPEL- UND SCHACHTELBARER STERILGUTKORB**
STACKABLE AND NESTABLE STERILE GOODS BASKET
PANIER GERBABLE POUR PRODUITS STÉRILES

(30) Priorität: 14.11.2016 DE 102016121723
(43) Veröffentlichungstag der Anmeldung: 16.05.2018
(73) Patentinhaber: HUPFER Metallwerke GmbH & Co. KG, 48653 Coesfeld (DE)
(72) Erfinder: LANWER, Thorsten, 48653 Coesfeld (DE)
(74) Vertreter: Andrejewski - Honke Patent- und Rechtsanwälte Partnerschaft mbB

(56) Entgegenhaltungen:
- DE-U1- 8 235 296
- GB-A- 1 331 152
- NL-C1- 1 004 306
- US-A- 3 752 352
- US-A- 3 923 187

## Beschreibung

Die Erfindung betrifft einen Sterilgutkorb gemäß dem Oberbegriff des Anspruches 1. Diese Merkmale ergeben sich aus US 3,923,187, welches Dokument einen Drahtkorb mit einstückig aus Flachmaterial gefertigten Klappbügeln beschreibt.

Ein weiterer Sterilgutkorb ist aus dem Prospekt "HUPFER®, Spezialist für Sterilgutlogistik - Körbe aus Chromnickelstahl", 2011 bekannt.

Der Sterilgutkorb ist bevorzugt aus einem rostfreien Edelstahldraht gebildet, weshalb er auch als Sterilgut-Gitterkorb bezeichnet wird.

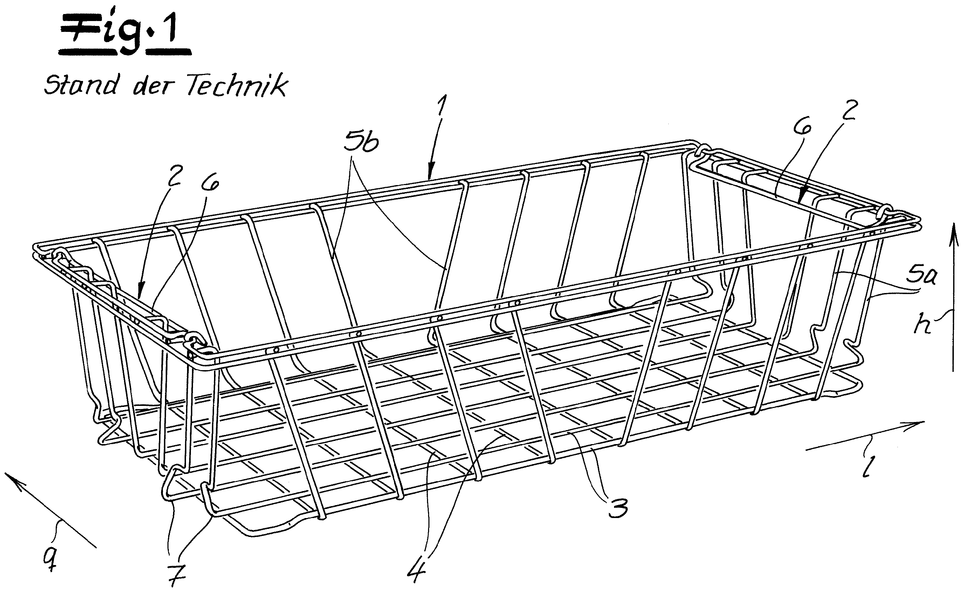

Der bekannte Sterilgutkorb (Fig. 1) weist bei einer rechteckigen Grundfläche (bezogen auf eine Projektion von oben) einen Boden mit in Richtung der langen Seiten verlaufenden Längsstreben und in Richtung der kurzen Seiten verlaufenden Querstreben auf, wobei die Längsstreben an den kurzen Seiten und die Querstreben an den langen Seite in Streben übergehen, welche entlang einer Höhe, also im Wesentlichen entlang einer Vertikalrichtung verlaufen. Diese entlang der Höhe verlaufenden Streben sind gegenüber der Senkrechten leicht schräg gestellt. Einerseits erweitert sich der Sterilgutkorb zu seinem oberen Rand, so dass dieser mit einem gleichartigen Sterilgutkorb gestapelt werden kann. Des Weiteren sind die entlang der Höhe verlaufenden Streben ausgehend von jeweils einer Mitte der entsprechenden langen Seite bzw. kurzen Seite leicht V-förmig schräggestellt, so dass die einzelnen Streben auch bei einem Ineinanderstapeln zunächst in einen Zwischenraum der entlang der Höhe verlaufenden Streben des darunterliegenden Korbes eingreifen können und sich nicht behindern.

Um gleichartige Sterilgutkörbe wahlweise auch übereinander stapeln zu können, sind an den kurzen Seiten der Grundfläche, also an den kurzen Seiten des Sterilgutkorbes Klappbügel angeordnet, welche vollständig aus Runddraht gebogen sind. Die Klappbügel weisen eine horizontale Halteleiste und davon abgewinkelte Enden auf, welche mit umgebogenen Ösen an den kurzen Seiten am oberen Rand des Sterilgutkorbes klappbar, das heißt drehbar befestigt sind. An der kurzen Seite sind die zunächst entlang der Höhe verlaufenden Streben am oberen Rand horizontal nach außen umgebogen, so dass sich dort ein gerader Flanschabschnitt ergibt. Des Weiteren weisen die entlang der Höhe verlaufenden Streben an den kurzen Seiten im Bereich des Bodens auch Ausformungen auf, welche als Vorsprünge in Richtung der langen Seiten vorstehen.

In einer ersten Klappposition hängt die horizontale Halteleiste der Klappbügel an der Außenseite des Sterilgutkorbes nach unten und ist insofern funktionslos. Es ist dann möglich, mehrere gleich ausgestaltete Sterilgutkörbe ineinanderzustapeln, wodurch eine erhebliche Platzersparnis erreicht werden kann.

Wenn dagegen die Klappbügel, welche auch als Griffe benutzt werden können, in die zweite Klappposition überführt werden, so liegen die Klappbügel an den entlang der Höhe verlaufenden Streben der zugeordneten kurzen Seite an, so dass dann bei dem Aufsetzen eines gleichartigen Sterilgutkorbes die entsprechenden entlang der Höhe verlaufenden Streben der kurzen Seiten und insbesondere die im Bereich des Bodens gebildeten Ausformungen blockiert werden und somit die übereinandergesetzten Sterilgutkörbe übereinandergestapelt werden können.

Entlang der Halteleisten ist keine Bewegung möglich, weil die beiden Sterilgutkörbe entlang der parallel zur kurzen Seite verlaufenden Querrichtung mit einem gewissen Spiel in einem Formfluss gehalten sind.

Wenn dagegen bei übereinandergestapelten Sterilgutkörben der oberste Sterilgutkorb an einer seiner kurzen Seiten angehoben wird, so kann dieser entlang der parallel zur langen Seite verlaufenden Längsrichtung über den darunterliegenden Sterilgutkorb gezogen werden.

Wenn der oberste Sterilgutkorb dann nicht mit großer Sorgfalt von einem Benutzer gehalten wird, besteht die Gefahr, dass die nicht mehr auf dem darunterliegenden Sterilgutkorb aufliegende in Zugrichtung hintere kurze Seite nach unten absackt und mit dem Füllgut des darunterliegenden Sterilgutkorbes in einen direkten Kontakt kommt. Es besteht dann die Gefahr einer Beschädigung und/oder einer Kontamination.

Aus der NL 1004306 C1 ist ein Sterilgutkorb bekannt, bei dem Klappbügel an den langen Seiten mit ihren Enden angelenkt sind, sich jedoch parallel zu der kurzen Seite erstrecken. Je nach Position der Klappbügel können mehrere Sterilgutkörbe entweder ineinander gestapelt oder übereinander gestapelt werden. Ein zuverlässiges Verschieben ohne ein Absacken ist nicht möglich.

GB 1 331 152 beschreibt einen weiteren Gitterkorb mit sich entlang seiner längeren Seitenkanten erstreckenden Klappbügeln.

Die US 3 752 352 A beschreibt eine Gitteranordnung, welche an zwei gegenüberliegenden Seiten offen ist und dort lediglich einen Klappbügel aufweist. Die beiden Klappbügel sind an einer langen Seite angelenkt und erstrecken sich davon ausgehend entlang der kurzen Seite. Abhängig von der Position der Klappbügel können mehrere dieser Gitteranordnungen entweder ineinander oder übereinander gestapelt werden.

Vor diesem Hintergrund liegt der vorliegenden Erfindung die Aufgabe zugrunde, ein einfaches und zuverlässiges Verschieben von gestapelten Sterilgutkörben entlang der Längsrichtung bei einer geringen mechanischen Belastung zu ermöglichen.

Gegenstand der Erfindung und Lösung der Aufgabe ist ein Sterilgutkorb gemäß Patentanspruch 1.

Die Form des Sterilgutkorbes und die Klappbügel sind so ausgeführt, dass bei einem Übereinanderstapeln mehrerer Sterilgutkörbe der oberste Sterilgutkorb angehoben und auf den horizontalen Halteleisten entlang einer Längsrichtung parallel zu den langen Seiten verschoben werden kann und dabei abgestützt bleibt.

Auf diese Weise kann verhindert werden, dass die in Zugrichtung hintere kurze Seite des Sterilgutkorbes unkontrolliert absackt und das enthaltene Füllgut beschädigt oder kontaminiert wird. Während gemäß dem Stand der Technik die an den kurzen Seiten angeordneten Klappbügel mit jeweils einer Halteleiste eine Abstützung in vertikaler Richtung bewirken und darüber hinaus die Sterilgutkörbe entlang der Halteleisten auch überhaupt nicht gegeneinander bewegt werden können, wird im Rahmen der Erfindung durch die Anordnung der Klappbügel an den langen Seiten den Halteleisten auch eine weitere Funktion nach Art von Leit- und Führungsschienen zugeordnet, wodurch sich eine besonders komfortable und zuverlässige Handhabung ergibt.

Gemäß einer bevorzugten Ausgestaltung der Erfindung ist vorgesehen, dass die Klappbügel in der zweiten Klappposition jeweils mit der horizontalen Halteleiste gegenüber entlang einer Höhe, das heißt im Wesentlichen in vertikaler Richtung verlaufenden Streben der zugeordneten langen Seite nach innen vorstehen. Besonders bevorzugt liegen die Halteleisten auch nicht an den entlang der Höhe verlaufenden Streben an, so dass ein ausreichender Freiraum verbleibt, um die Sterilgutkörbe bei der beschriebenen Schiebebewegung entlang der Längsrichtung ohne Einklemmen sicher zu führen.

In an sich bekannter Weise können die Halteleisten aus Runddraht gebildet sein.

Um im Rahmen der Erfindung eine sichere Abstützung auch bei der beschriebenen Schiebebewegung sicherzustellen, erstrecken sich die Halteleisten über zumindest 80 % der Länge der langen Seiten, so dass auch kurz vor einem vollständigen Abnehmen eines oberen Sterilgutkorbes nach einer Schiebebewegung ein Absacken der in Zugrichtung hinteren kurzen Seite vermieden wird.

Um des Weiteren ein Verhaken zu vermeiden und gleichartige Sterilgutkörbe auch durch eine Schiebebewegung leicht übereinanderstapeln zu können, sind die Halteleisten gemäß einer bevorzugten Weiterbildung der Erfindung in Längsrichtung gesehen an ihren Enden nach unten abgeschrägt bzw. umgebogen.

Gemäß der Erfindung sind die Klappbügel jeweils mit einer Vielzahl von Laschen, also zumindest zwei Laschen, klappbar an dem oberen Rand befestigt. Während gemäß dem Stand der Technik die gesamten Haltebügel mit jeweils einer Halteleiste und Befestigungsenden aus einem einzigen Runddraht gebogen sind, sind gemäß der vorliegenden Erfindung separate Laschen vorgesehen, welche fest mit der vorzugsweise aus Runddraht gebildeten Halteleiste verbunden sind. Es ist sodann auch möglich, dass mehr als zwei, beispielsweise zwischen drei und sechs und insbesondere vier Laschen vorgesehen sind, so dass auch eine ausreichende Tragkraft der Klappbügel sichergestellt werden kann. Dabei ist auch zu berücksichtigen, dass gerade bei einer Schiebebewegung lokal relativ große Kräfte abzustützen sind.

Die Laschen können auf besonders einfache Weise aus Blech gebildet sein, wobei jedoch auch andere Materialien, wie beispielsweise Kunststoff, in Betracht kommen.

Die Laschen sind mit einem Ösenabschnitt am oberen Rand drehbar befestigt und über einen Verbindungsschenkel an die jeweils zugeordnete Halteleiste angeschlossen. Der Ösenabschnitt kann gerade bei einer Ausgestaltung der Laschen aus Blech auf einfache Weise gebogen werden. Gegebenenfalls können dann die aus Blech gebogenen Ösenabschnitte durch eine Verschweißung fixiert werden, damit diese sich nicht wieder aufbiegen können. Die Befestigung des Verbindungsschenkels an der Halteleiste kann beispielsweise durch Schweißen erfolgen, wobei Punktschweißungen ausreichend sein können.

Damit im Rahmen der erfindungsgemäßen Ausgestaltung die Verbindungsschenkel eine Schiebebewegung nicht blockieren und die Klappbügel auch leicht vollständig nach außen geklappt werden können, erstreckt sich in der zweiten Klappposition der Ösenabschnitt ausgehend von dem Verbindungsschenkel nach oben, wobei der Verbindungsschenkel auch an einer Unterseite der horizontalen Halteleiste befestigt ist. Gerade bei einer solchen Anordnung wirken bei der Benutzung nur geringe Kräfte auf die Verbindung zwischen Verbindungsschenkel und Halteleiste.

Um das Gewicht eines darüber angeordneten Sterilgutkorbes sicher aufnehmen und ableiten zu können, sind die Laschen gemäß einer bevorzugten Weiterbildung der Erfindung jeweils im Bereich einer entlang der Höhe verlaufenden Strebe der zugeordneten langen Seite angeordnet, wobei sich die Laschen in der zweiten Klappposition gegen die jeweils zugeordnete Strebe abstützen.

Konkret kann insbesondere vorgesehen sein, dass der Ösenabschnitt eine Unterbrechung und der Verbindungsschenkel eine an die Unterbrechung anschließende Einbuchtung aufweisen. Die zugeordnete entlang der Höhe verlaufende Strebe befindet sich dann im Bereich der Unterbrechung sowie der daran anschließenden Einbuchtung, wobei sich die Laschen dann in der zweiten Klappposition jeweils mit einem Rand der Einbuchtung an der zugeordneten Strebe abstützen.

Bei dem erfindungsgemäßen Sterilgutkorb ist vorzugsweise ein Boden aus parallel zu den langen Seiten verlaufenden Längsstreben und parallel zu den kurzen Seiten verlaufenden Querstreben gebildet, wobei der Abstand von außenliegenden Längsstreben kleiner ist als der Abstand der horizontalen Haltestreben in der zweiten Klappposition.

Besonders bevorzugt sind die außenliegenden Längsstreben bei einem Übereinanderstapeln mit wenig Spiel zwischen den horizontalen Halteleisten eines darunterliegenden Sterilgutkorbes angeordnet, so dass dann die zuvor beschriebene Schiebebewegung geführt ist. Das Spiel kann beispielsweise zwischen 1 mm und 10 mm betragen und wird zweckmäßigerweise so ausgewählt, so dass geringe Maßabweichungen nicht zu einem Klemmen führen, andererseits jedoch die außenliegenden Längsstreben an den horizontalen Halteleisten zuverlässig geführt werden können.

Im Rahmen der beschriebenen Ausgestaltung verlaufen die Querstreben oberhalb der außenliegenden Längsstreben und auch in Querrichtung über die Längsstreben hinaus, so dass dann bei einem Übereinanderstapeln mehrerer Sterilgutkörbe der obere Sterilgutkorb mit den über die außenliegenden Längsstreben herausstehenden Querstreben auf den Halteleisten aufliegt. Beispielsweise kann im Rahmen der Erfindung vorgesehen sein, dass die Querstreben lediglich oberhalb der außenliegenden Längsstreben aber unterhalb der übrigen Längsstreben verlaufen. Besonders bevorzugt ist vorgesehen, dass die Querstreben in Querrichtung gesehen vor dem oberen Rand enden.

Die Erfindung wird im Folgenden anhand von exemplarischen Zeichnungen erläutert. Es zeigen:
- Fig.1: einen Sterilgutkorb gemäß dem Stand der Technik,
- Fig. 2: zwei übereinandergestapelte erfindungsgemäße Sterilgutkörbe,
- Fig. 3: die Sterilgutkörbe gemäß der Fig. 2 bei einer Verschiebung entlang einer Längsrichtung,
- Fig. 4: eine Detailansicht eines Sterilgutkorbes mit einem Klappbügel in einer ersten Klappposition, welche ein Ineinanderstapeln ermöglicht,
- Fig. 5: eine Detailansicht von zwei übereinandergestapelten erfindungsgemäßen Sterilgutkörben, wobei sich der dargestellte Klappbügel in einer zweiten Klappposition befindet.

Die Fig. 1 zeigt einen bekannten Sterilgutkorb gemäß dem Stand der Technik mit einer zwei lange Seiten und zwei kurze Seiten aufweisenden rechteckigen Grundfläche.

An einem oberen Rand 1 sind an den kurzen Seiten zwei Klappbügel 2 angeordnet.

Der Sterilgutkorb weist einen Boden aus in Richtung der langen Seiten, das heißt parallel zu den langen Seiten verlaufenden Längsstreben 3 und parallel zu den kurzen Seiten verlaufenden Querstreben 4 auf. Entsprechend werden der rechteckigen Grundfläche des sterilen Gutkorbes auch die Begriffe Längsrichtung I und Querrichtung q zugeordnet.

Die Längsstreben 3 gehen an den kurzen Seiten und die Querstreben 4 an den langen Seiten in Streben 5a, 5b über, welche entlang der Höhe h des Sterilgutkorbes verlaufen.

Diese Streben 5a, 5b verlaufen jedoch nicht genau senkrecht. Einerseits sind diese entlang der Höhe h verlaufenden Streben 5a, 5b leicht nach außen und andererseits von einer Mitte der jeweiligen Seite weg schräggestellt.

Die Schrägstellung ermöglicht, dass gleiche Sterilgutkörbe auch ineinandergestapelt werden können. Durch die Schrägstellung ist der durch die Längsstreben 3 und Querstreben 4 gebildete Boden etwas kleiner als die Fläche des Sterilgutkorbes am oberen Rand 1, wobei durch die Verkippung von einer Mitte der jeweiligen Seite weg auch die Streben 5a, 5b jeweils in den Freiraum zwischen den Streben 5a, 5b eines darunterliegenden Sterilgutkorbes eingesetzt werden können.

Um zumindest zwei gleichartige Sterilgutkörbe wahlweise ineinander oder übereinander zu stapeln, sind am oberen Rand 1 die Klappbügel 2 vorgesehen, welche sich in der Fig. 1 in einer zweiten Klappposition befinden. In der zweiten Klappposition liegt eine horizontale Halteleiste 6 im Bereich des oberen Randes 1 an den entlang der Höhe h verlaufenden Streben 5a der zugeordneten kurzen Seite an, wobei die entsprechenden Streben 5a zumindest teilweise im Bereich des Bodens Ausformungen 7 aufweisen, welche entlang der Längsrichtung I vorstehen.

Wenn sich - wie in Fig. 1 dargestellt - die Klappbügel 2 in der zweiten Klappposition befinden, liegen bei einem Übereinanderstapeln die Ausformungen 7 auf der jeweils darunterliegenden Halteleiste 6 eines weiteren Sterilgutkorbes auf. Abgesehen von einem gewissen Spiel liegt zwischen den Sterilgutkörben ein Formschluss vor, wobei eine Bewegung der Sterilgutkörbe entlang der in Querrichtung q verlaufenden Halteleisten 6 auch dann nicht möglich ist, wenn der oberste Sterilgutkorb angehoben wird. Auch dann schlagen die Ausformungen 7 im Bereich des oberen Randes 1 an den angrenzenden entlang der Höhe h verlaufenden Streben 5a der kurzen Seite an.

Wenn dagegen versucht wird, bei übereinandergestapelten Sterilgutkörben einen obersten Sterilgutkorb entlang der Längsrichtung I von einem darunterliegenden Sterilgutkorb herunterzuziehen, so ist dann zumindest die in Zugrichtung hintere kurze Seite nicht nach unten abgestützt, so dass der obere Sterilgutkorb dort unkontrolliert absacken kann. Es besteht die Gefahr, dass die in dem unteren Sterilgutkorb aufgenommenen Sterilgüter beschädigt und/oder konterminiert werden.

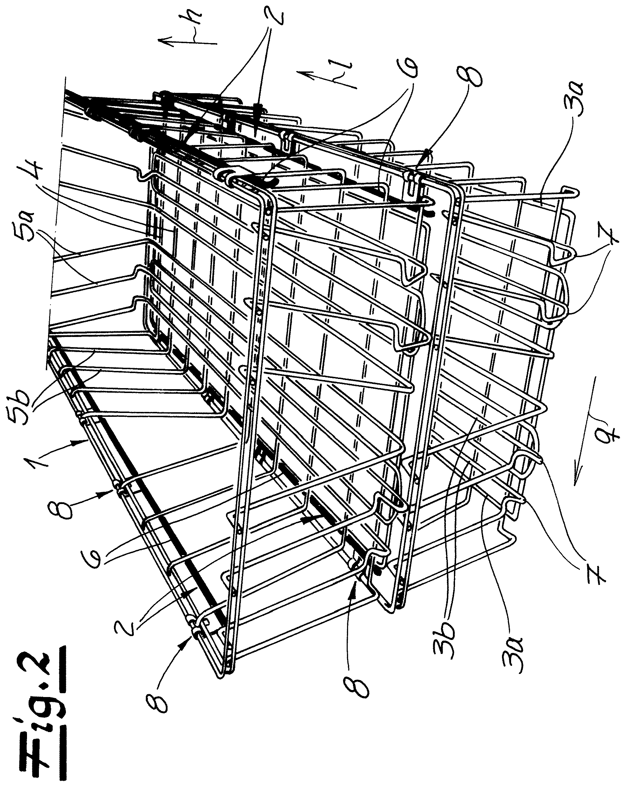

Um diesen Nachteil zu vermeiden, ist bei dem erfindungsgemäßen Sterilgutkorb gemäß der Fig. 2 vorgesehen, dass die Klappbügel 2 an den langen Seiten angeordnet sind und als zusätzliche Funktion eine Schiebeführung für eine Bewegung entlang der Längsrichtung I bilden.

Bei einer ansonsten vergleichbaren Ausgestaltung ergibt sich gegenüber dem bekannten Stand der Technik eine völlig andere Funktion und Konstruktion der Klappbügel 2.

Die Fig. 2 zeigt zwei übereinandergestapelte Sterilgutkörbe, wobei sich die an den langen Seiten angeordneten Klappbügel 2 bei dem unteren Sterilgutkorb in der zweiten Klappposition und bei dem oberen Sterilgutkorb in der ersten Klappposition befinden.

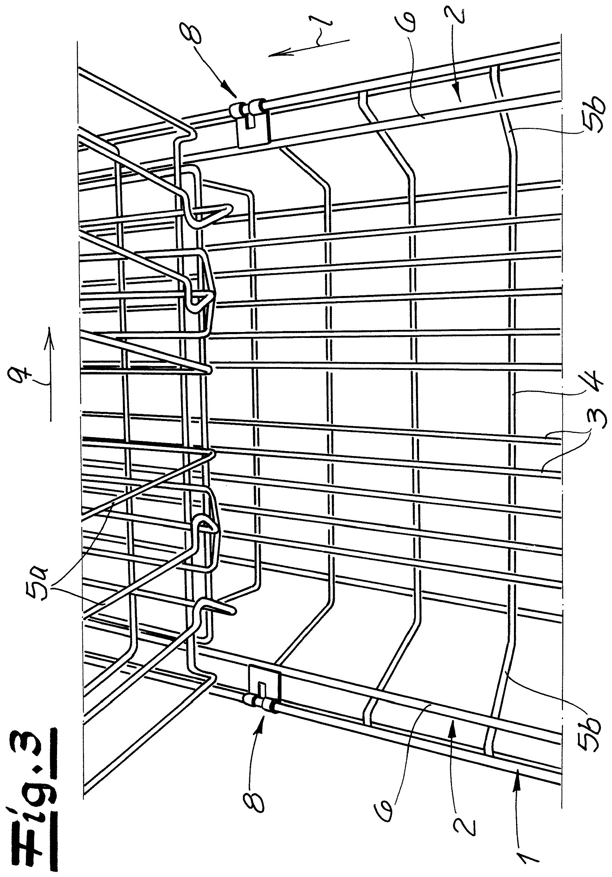

In der Fig. 3 ist dargestellt, dass der obere Sterilgutkorb leicht und zuverlässig entlang der Längsrichtung I von dem unteren Sterilgutkorb heruntergezogen werden kann, ohne dass das in dem unteren Sterilgutkorb aufgenommene Sterilgut beschädigt oder kontaminiert werden kann. Konkret ist der obere Sterilgutkorb auch bei einer Schiebebewegung durch die Halteleisten 6 der Klappbügel 2 nach unten zuverlässig abgestützt. Wie nachfolgend noch im Detail beschrieben, kann aufgrund der gewählten Ausgestaltung der obere Sterilgutkorb auch nicht seitlich herunterrutschen.

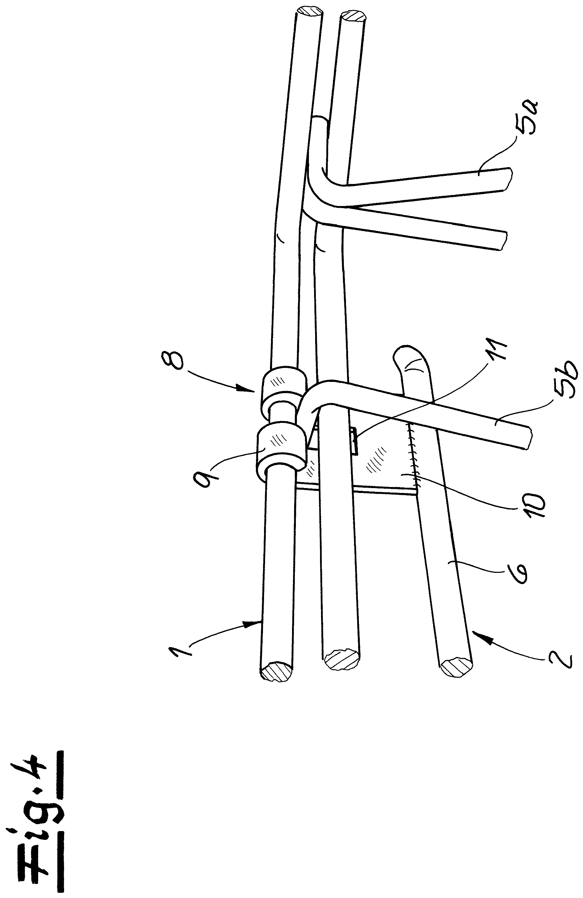

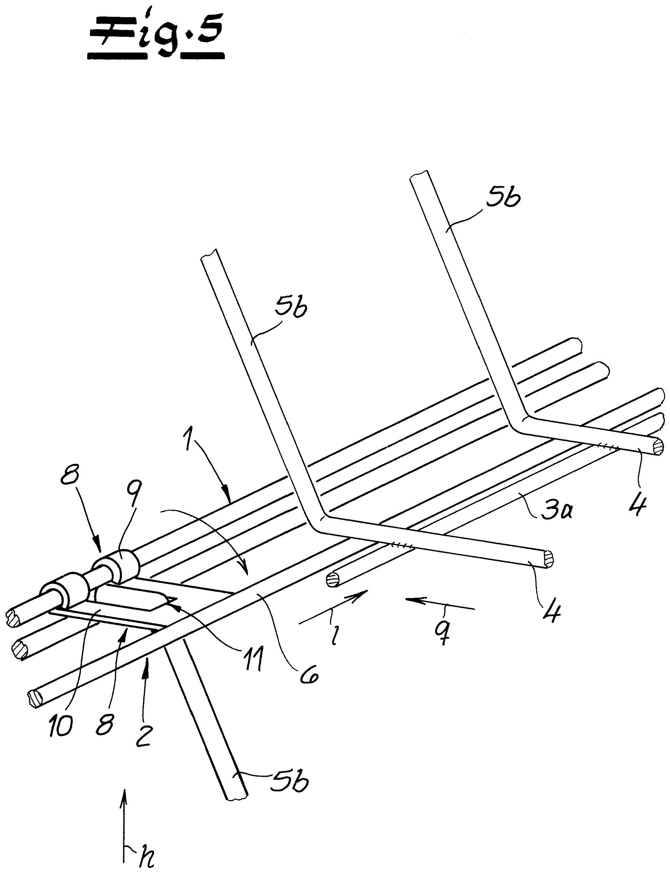

Die Fig. 4 und 5 zeigen Detailansichten, wobei in der Fig. 4 der Klappbügel 2 sich in der ersten Klappposition befindet und an der Außenseite des Sterilgutkorbes herunterhängt. Die Fig. 5 zeigt dagegen eine Detailansicht entsprechend der in Fig. 3 dargestellten Anordnung.

Insbesondere aus den Fig. 3 bis 5 ist ersichtlich, dass die Klappbügel 2 jeweils aus der von einem Runddraht gebildeten Halteleiste 6 und in dem Ausführungsbeispiel vier Laschen 8 gebildet sind. Die Laschen 8 ermöglichen einerseits eine zuverlässige drehbare Befestigung der Klappbügel 2 und dienen auch im Hinblick auf die Halteleisten 6 als eine Art Abstandhalter in Bezug auf die entlang der Höhe h verlaufenden Streben 5b der jeweils angrenzenden langen Seite. Die Laschen 8 können beispielsweise auf einfache Weise aus Blech gebildet sein und weisen einen Ösenabschnitt 9 und einen Verbindungsschenkel 10 auf. Der Ösenabschnitt 9 schließt oberhalb des Verbindungsschenkels 10 an, wobei der Verbindungsschenkel 10 auch gemäß der Fig. 5 an der Unterseite der Halteleiste 6 befestigt, insbesondere angeschweißt ist, so dass der obere Bereich der Halteleiste 6 freiliegt und eine klemmfreie Schiebebewegung eines aufliegenden Sterilgutkorbes möglich ist.

Insbesondere in den Fig. 4 und 5 ist zu erkennen, dass der Ösenabschnitt 9 eine Unterbrechung aufweist, wobei der Verbindungsschenkel 10 eine an die Unterbrechung anschließende Einbuchtung 11 aufweist.

Jede Lasche 8 ist im Bereich einer entlang der Höhe h verlaufenden Strebe 5b der zugeordneten langen Seite angeordnet, wobei sich die Unterbrechung des Ösenabschnittes 9 genau an der Position der zugeordneten Strebe 5b befindet. Entsprechend können sich die Laschen 8 in der zweiten Klappposition jeweils mit einem Rand der Einbuchtung 11 an der zugeordneten Strebe 5b abstützen (Fig. 5). Durch diese Abstützung und die Verteilung der Last auf vier Laschen 8 pro langer Seite kann dauerhaft eine hohe Stabilität sichergestellt werden.

In den Fig. 3 und 5 ist zu erkennen, dass die beiden außenliegenden Längsstreben 3a des erfindungsgemäßen Sterilgutkorbes einen kleineren Abstand aufweisen als die horizontalen Halteleisten 6 in der zweiten Klappposition. Bei einem Übereinanderstapeln gleich ausgebildeter Sterilgutkörbe befinden sich die beiden außenliegenden Längsstreben 3a also gemäß der Fig. 5 innerhalb der beiden Halteleisten 6, wobei vorzugsweise ein geringes Spiel von beispielsweise 1 mm bis 10 mm verbleibt. Durch eine solche Ausgestaltung kann eine besonders zuverlässige aber auch klemmfreie Führung bei einer Schiebebewegung entlang der Längsrichtung I sichergestellt werden.

Es ist auch zu erkennen, dass die Querstreben 4 oberhalb der außenliegenden Längsstreben 3a verlaufen und in Querrichtung q über die außenliegenden Längsstreben 3a hinausgehen. Die Querstreben 4 enden in Querrichtung q gesehen vor dem oberen Rand 1, der in dem Ausführungsbeispiel von einem umlaufenden Runddraht gebildet ist, das heißt die Querstreben 4 gehen dort in einem Radius in die entlang der Höhe h verlaufenden Streben 5b der langen Seiten über.

In dem konkreten Ausführungsbeispiel sind lediglich die außenliegenden Längsstreben 3a unterhalb der Querstreben 4 angeordnet, während die weiteren, innenliegenden Längsstreben 3b sich oberhalb der Querstreben 4 befinden. Eine solche Ausgestaltung ist im Rahmen des dargestellten Ausführungsbeispiels zweckmäßig, weil die Längsstreben 3a, 3b ein engeres Rastermaß, das heißt, einen geringeren Abstand als die Querstreben 4 aufweisen, so dass durch die in dem mittleren Bereich des Boden obenliegenden Längsstreben 3b eine möglichst ebene Fläche bereitgestellt wird.

## Patentansprüche

1. Sterilgutkorb mit einer zwei lange Seiten und zwei kurze Seiten aufweisenden rechteckigen Grundfläche und mit zwei an einem oberen Rand (1) angeordneten Klappbügeln (2), welche in einer ersten Klappposition ein Ineinanderstapeln und in einer zweiten Klappposition mit jeweils einer horizontalen Halteleiste (6) ein Übereinanderstapeln gleichartiger Sterilgutkörbe ermöglichen, wobei die Klappbügel (2) an den langen Seiten angeordnet sind, wobei die Klappbügel (2) jeweils mit einer Vielzahl von Laschen (8) klappbar an dem oberen Rand (1) befestigt sind und wobei die Laschen (8) mit einem Ösenabschnitt (9) am oberen Rand (1) drehbar befestigt sind und über einen Verbindungsschenkel (10) an die Halteleiste (6) angeschlossen sind, **dadurch gekennzeichnet, dass** in der zweiten Klappposition der Ösenabschnitt (9) sich von dem Verbindungsschenkel (10) ausgehend nach oben erstreckt und der Verbindungsschenkel (10) an einer Unterseite der horizontalen Halteleiste (6) befestigt ist.

2. Sterilgutkorb nach Anspruch 1, **dadurch gekennzeichnet, dass** die Klappbügel (2) in der zweiten Klappposition jeweils mit der horizontalen Halteleiste (6) gegenüber entlang einer Höhe (h) zwischen einem Boden und dem oberen Rand (1) verlaufenden Streben (5b) der zugeordneten langen Seite nach innen vorstehen.

3. Sterilgutkorb nach Anspruch 2, **dadurch gekennzeichnet, dass** die Laschen (8) jeweils im Bereich einer entlang der Höhe (h) verlaufenden Strebe (5b) der zugeordneten langen Seite angeordnet sind und sich in der zweiten Klappposition gegen diese jeweils zugeordnete Strebe (5b) abstützen.

4. Sterilgutkorb nach Anspruch 3, **dadurch gekennzeichnet, dass** jeder Ösenabschnitt (9) eine Unterbrechung und jeder Verbindungsschenkel (10) eine an die Unterbrechung anschließende Einbuchtung (11) aufweisen, wobei sich die Laschen (8) in der zweiten Klappposition jeweils mit einem Rand der Einbuchtung (11) an der zugeordneten Strebe (5b) abstützen.

5. Sterilgutkorb nach einem der Ansprüche 1 bis 4, **dadurch gekennzeichnet, dass** die Halteleisten (6) aus einem Runddraht gebildet sind.

6. Sterilgutkorb nach einem der Ansprüche 1 bis 5, **dadurch gekennzeichnet, dass** die Halteleisten (6) sich über zumindest 80 % der Länge der langen Seiten erstrecken.

7. Sterilgutkorb nach einem der Ansprüche 1 bis 6, **dadurch gekennzeichnet, dass** die Laschen (8) aus Blech gebildet sind.

8. Sterilgutkorb nach einem der Ansprüche 1 bis 7, **dadurch gekennzeichnet, dass** ein Boden aus in Richtung der langen Seiten verlaufenden Längsstreben (3a, 3b) und in Richtung der kurzen Seiten verlaufenden Querstreben (4) gebildet ist, wobei der Abstand von außenliegenden Längsstreben (3a) kleiner ist als der Abstand der horizontalen Halteleisten (6) in der zweiten Klappposition und dass die Querstreben (4) oberhalb der außenliegenden Längsstreben (3a) verlaufen und in Querrichtung (9) über die Längsstreben (3a, 3b) hinausstehen.

9. Sterilgutkorb nach Anspruch 8, **dadurch gekennzeichnet, dass** die Querstreben (4) in Querrichtung (9) gesehen vor dem oberen Rand (1) enden.

## Claims

1. A sterile goods basket with a rectangular base comprising two long sides and two short sides and with two folding handles (2) arranged on an upper edge (1), which in a first folding position allow identical sterile goods baskets to be stacked inside one another and in a second folding position in conjunction with a respective horizontal retaining bar (6), allow identical sterile goods baskets to be stacked on top of each other, the folding handles (2) being arranged on the long sides, wherein the folding handles (2) are each foldably attached by a plurality of straps (8) to the upper edge (1) and wherein the straps (8) are rotatably fastened by a loop portion (9) to the upper edge (1) and connected via a connecting shank (10) to the retaining bar (6), **characterised in that** in the second folding position the loop portion (9) extends upwards starting from the connecting shank (10) with the connecting shank (10) being fastened to an underside of the horizontal retaining bar (6).

2. The sterile goods basket according to claim 1, **characterised in that** the folding handles (2), in the second folding position, each protrude with the horizontal retaining bar (6) towards the inside relative to struts (5b) of the associated long side, which extend along a height (h) between a bottom and the upper edge (1).

3. The sterile goods basket according to claim 2, **characterised in that** the straps (8) are each arranged in the region of a strut (5b) of the associated long side extending along the height (h) and, in the second folding position, are supported against this respectively associated strut (5b).

4. The sterile goods basket according to claim 3, **characterised in that** each loop portion (9) comprises an interruption and each connecting shank (10) comprises an indentation (11) following the interruption, wherein the straps (8), in the second folding position, are supported with an edge of the indentation (11) against the associated strut (5b).

5. The sterile goods basket according to one of claims 1 to 4, **characterised in that** the retaining bars (6) are formed from a round wire.

6. The sterile goods basket according to one of claims 1 to 5, **characterised in that** the retaining bars (6) extend over at least 80% of the length of the long sides.

7. The sterile goods basket according to one of claims 1 to 6, **characterised in that** the straps (8) are formed from sheet metal.

8. The sterile goods basket according to one of claims 1 to 7, **characterised in that** a bottom is formed from longitudinal struts (3a, 3b) extending in direction of the long sides and cross struts (4) extending in direction of the short sides, wherein the distance of external longitudinal struts (3a) is smaller than the distance of the horizontal retaining bars (6) in the second folding position and **in that** the cross struts (4) extend above the external longitudinal struts (3a) and in transverse direction (9) protrude beyond the longitudinal struts (3a, 3b).

9. The sterile goods basket according to claim 8, **characterised in that** the cross struts (4), when viewed in transverse direction (9), end before the upper edge (1).

## Revendications

1. Panier pour produits stériles avec une surface de base rectangulaire présentant deux côtés longs et deux côtés courts et avec deux anses rabattables (2) disposés sur un bord supérieur (1), lesquels permettent, dans une première position de rabattement, un empilement les uns dans les autres, et dans une deuxième position de rabattement avec respectivement une baguette de retenue horizontale (6), un empilement les uns sur les autres de paniers pour produits stériles du même genre,
dans lequel les anses rabattables (2) sont respectivement disposés au niveau des côtés longs, dans lequel les anses rabattables (2) sont respectivement fixés de façon rabattable avec une pluralité d'attaches (8) au bord supérieur (1), et dans lequel les attaches (8) sont fixées de manière rotative au bord supérieur (1) avec une partie en boucle (9) et sont reliées via une branche de liaison (10) à la baguette de retenue (6),
**caractérisé en ce que**, dans la deuxième position de rabattement, la partie en boucle (9) s'étend vers le haut en partant de la branche de liaison (10), et la branche de liaison (10) est fixée à un dessous de la baguette de retenue horizontale (6).

2. Panier pour produits stériles selon la revendication 1, **caractérisé en ce que**, dans la deuxième position de rabattement, les anses rabattables (2) font respectivement saillie vers l'intérieur avec la baguette de retenue horizontale (6) par rapport à des entretoises (5b), s'étendant le long d'une hauteur (h) entre un fond et le bord supérieur (1), du côté long associé.

3. Panier pour produits stériles selon la revendication 2, **caractérisé en ce que** les attaches (8) sont respectivement disposées dans la zone d'une entretoise (5b), s'étendant le long de la hauteur (h), du côté long associé, et sont en appui sur cette entretoise (5b) respectivement associée dans la deuxième position de rabattement.

4. Panier pour produits stériles selon la revendication 3, **caractérisé en ce que** chaque partie en boucle (9) présente une interruption et chaque branche de liaison (10) présente un évidement (11) en jonction avec l'interruption, dans lequel, dans la deuxième position de rabattement, les attaches (8) sont respectivement en appui avec un bord de l'évidement (11) sur l'entretoise (5b) associée.

5. Panier pour produits stériles selon l'une des revendications 1 à 4, **caractérisé en ce que** les baguettes de retenue (6) sont formées par un fil rond.

6. Panier pour produits stériles selon l'une des revendications 1 à 5, **caractérisé en ce que** les baguettes de retenue (6) s'étendent sur au moins 80% de la longueur des côtés longs.

7. Panier pour produits stériles selon l'une des revendications 1 à 6, **caractérisé en ce que** les attaches (8) sont formées en tôle.

8. Panier pour produits stériles selon l'une des revendications 1 à 7, **caractérisé en ce qu'**un fond est formé par des entretoises longitudinales (3a, 3b) s'étendant le long des côtés longs et des entretoises transversales (4) s'étendant en direction des côtés courts, dans lequel l'écartement d'entretoises longitudinales (3a) situées à l'extérieur est inférieur à l'écartement des baguettes de retenue horizontales (6) dans la deuxième position de rabattement, et que les entretoises transversales (4) s'étendent au-dessus des entretoises longitudinales (3a) situées à l'extérieur et font saillie en direction transversale (9) par rapport aux entretoises longitudinales (3a, 3b).

9. Panier pour produits stériles selon la revendication 8, **caractérisé en ce que** les entretoises transversales (4) se terminent devant le bord supérieur (1) en vue en direction transversale (9).
